# EUROPEAN PATENT SPECIFICATION

(11) **EP 3 706 682 B1**
(45) Date of publication and mention of the grant of the patent: **14.10.2026**
(21) Application number: 18836648.8
(22) Date of filing: 12.12.2018
(51) Int. Cl.: A61B 18/22, G02B 6/02, G02B 6/42, A61F 9/008, A61B 18/20

(54) **THERMALLY ROBUST LASER PROBE ASSEMBLY**
THERMISCH ROBUSTE LASERSONDENANORDNUNG
ENSEMBLE SONDE LASER THERMIQUEMENT ROBUSTE

(30) Priority: 12.12.2017 US 201762597550 P; 14.12.2017 US 201762598653 P; 26.01.2018 US 201862622299 P; 15.02.2018 US 201862630865 P
(43) Date of publication of application: 16.09.2020
(73) Proprietor: Alcon Inc., 1701 Fribourg (CH)
(72) Inventor: DIAO, Chenguang, Lake Forest, California 92630 (US); MIRSEPASSI, Alireza, Lake Forest, California 92630 (US); COOK, Christopher, Lake Forest, California 92630 (US)
(74) Representative: Murgitroyd & Company
(86) International application number: PCT/IB2018/059978
(87) International publication number: WO 2019/116284

(56) References cited:
- US-A1- 2002 045 811
- US-A1- 2011 122 366
- US-A1- 2014 194 862
- US-A1- 2015 366 432

## Description

### TECHNICAL FIELD

The present disclosure relates generally to methods for manufacturing a thermally robust laser probe assembly.

### BACKGROUND

A laser probe assembly may be used during a number of different procedures and surgeries. As an example, a laser probe assembly may be used during retinal laser surgeries in order to seal retinal tears, among other things. Laser light is typically transmitted from a laser source through an optical fiber cable. The optical fiber cable proximally terminates in a laser connector, which connects to the laser source, and distally terminates in a probe assembly that is manipulated by the surgeon. Note that, herein, a distal end of a component refers to the end that is closer to a patient's body, or where the laser light is emitted out of the laser probe. On the other hand, the proximal end of the component refers to the end that is facing away from the patient's body or in proximity to, for example, the laser source.

The probe assembly comprises a hand-piece coupled to a cannula that is partly inserted in a patient's eye. The optical fiber cable extends through the hand-piece and the cannula to transmit laser light onto the patient's retina. A lens may also be used to collimate and project the laser beams propagated by the optical fiber on the patient's retina for increased performance. Typically, the lens is placed in front of the optical fiber and is attached to the cannula.

In certain cases, the optical fiber cable houses more than one optical fiber, enabling the laser probe assembly to deliver more than one photocoagulation beam at the same time. For example, in certain cases, the optical fiber cable may house four optical fibers or a multi-core optical fiber. In such cases, due to the high power throughput in a confined space (e.g., within the cannula), the cannula and the lens may experience excessive heat when blood or other dark materials exist in front of or at least partially block or touch the tip of the cannula or the lens. In some cases, the excessive heat is created because the laser beams propagated by the optical fibers are reflected back by the blood or the dark material onto the lens, the cannula, and the adhesive bonding between the lens and the cannula. This overheating and thermal run-away results in the cannula and the lens melting and also causing the lens to detach from the cannula.
Reference is made to the documents US2011/122366, US2002/045811, US2014/194862 and US2015/366432 which have been cited as relating to the state of the art.

### BRIEF SUMMARY

It will be appreciated that the scope of the invention is in accordance with the claims. Accordingly, there is provided a system and method as defined in the claims. The specification may also include arrangements outside the scope of the claims provided as background and to assist in understanding the invention.

The present disclosure relates generally to methods for manufacturing a thermally robust laser probe assembly.

Produced by the method is a probe assembly comprising a cannula through which one or more optical fibers extend at least partially for transmitting laser light from a laser source to a target location. The probe assembly further comprises a lens housed in the cannula and a protective component at a distal end of the cannula, wherein the lens is positioned between the one or more optical fibers and the protective component, and wherein the distal end of the cannula is sealed at a sealing location of the probe assembly.

The method uses a sealant application system comprising a stage machine comprising a mount configured to hold a sealant applicator comprising a wire, wherein the stage machine is configured to position the wire at a sealing location at a distal end of a cannula of a probe assembly. The sealant application system also comprises a cannula holder comprising a groove for holding the cannula and an actuator configured to rotate the cannula once the stage machine has positioned the wire at the sealing location such that sealant on the wire is able to be applied to the sealing location.

Embodiments of the present invention provide a method of manufacturing a probe assembly. The method comprises positioning a wire coated with sealant at a sealing location at a distal end of a cannula of the probe assembly. The method further comprises rotating the cannula to apply the sealant to the sealing location.

The following description and the related drawings set forth in detail certain illustrative features of one or more embodiments.

The invention is defined by the appended claims.

### BRIEF DESCRIPTION OF THE DRAWINGS

The appended figures depict certain aspects of the one or more embodiments of the present invention and are therefore not to be considered limiting of the scope of this disclosure.
FIG. 1A illustrates a probe assembly comprising a hand-piece and a cannula in accordance with a particular embodiment of the present invention.
FIG. 1B illustrates a cross-sectional view of the tip of the cannula of FIG. 1A.
FIG. 2A illustrates a cross-sectional view of a protective component that is placed at the tip of a cannula in accordance with a particular embodiment of the present invention.

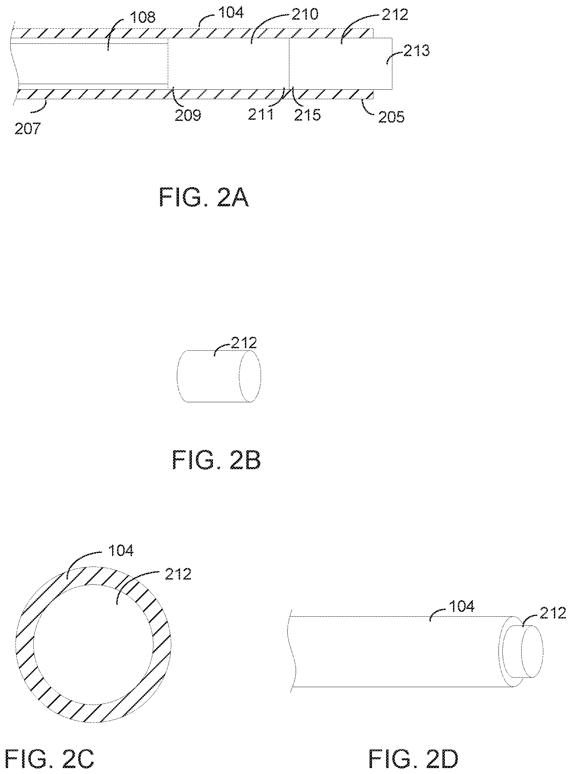

**FIG. 2A** illustrates a cross-sectional view of a protective component that is placed at the tip of a cannula in accordance with a particular embodiment of the present invention.
**FIG. 2B** illustrates a three-dimensional view of the protective component of **FIG. 2A****.**
**FIG. 2C** illustrates a front view of the tip of the cannula shown in **FIG. 2A****.**
**FIG. 2D** illustrates a three-dimensional view of the tip of the cannula shown in **FIG. 2A****.**

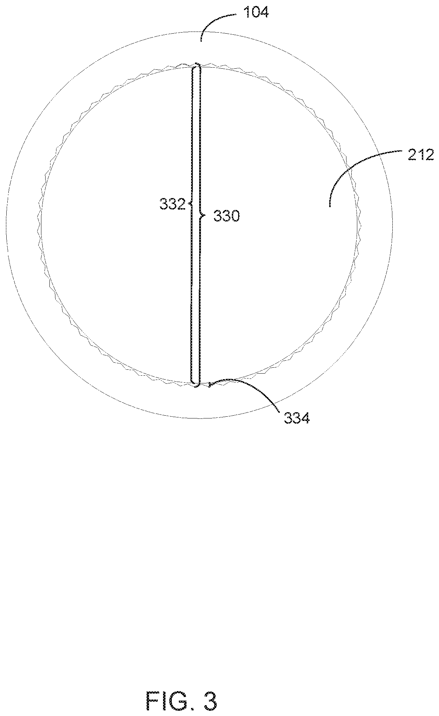

**FIG. 3** illustrates an example gap between the inner surface of the distal end of a cannula and the outer surface of the distal end of a protective component housed by the cannula.

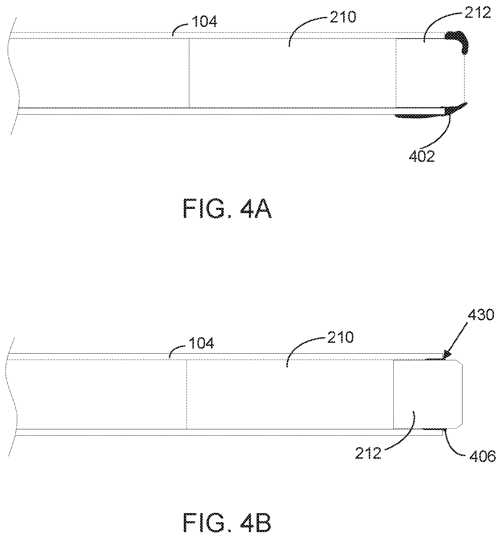

**FIG. 4A** illustrates an example of a contaminated protective component.
**FIG. 4B** illustrates a cross-sectional view of a sealed distal end of a cannula in accordance with a particular embodiment of the present invention.

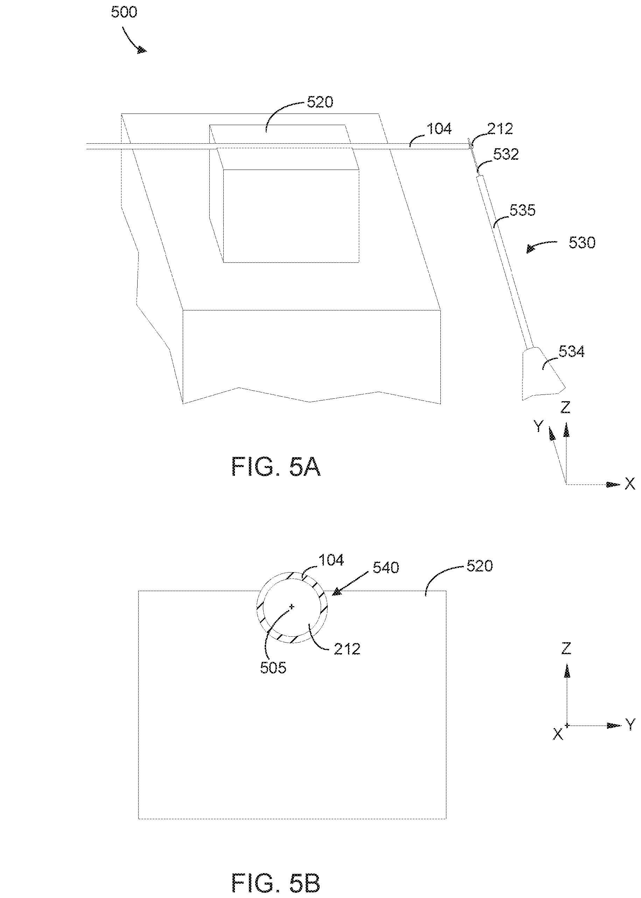

**FIG. 5A** illustrates a sealant application system in accordance with a particular embodiment of the present invention.
**FIG. 5B** illustrates a front view of a cannula holder holding a cannula in a u-shaped groove in accordance with a particular embodiment of the present invention.

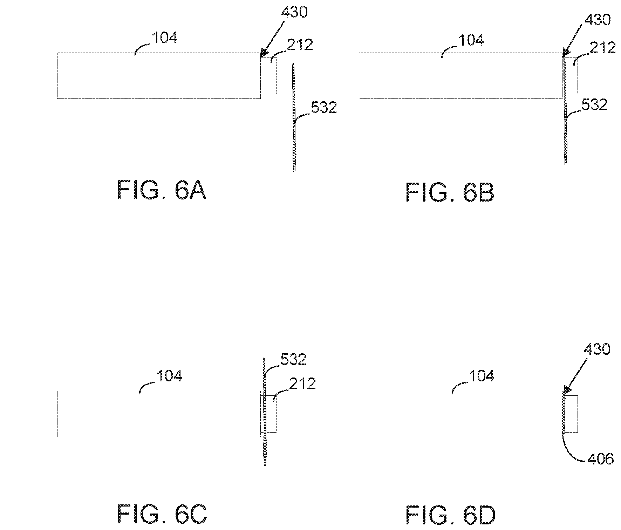

**FIG. 6A-6D** illustrate stages of a sealant application procedure in accordance with a particular embodiment of the present invention.

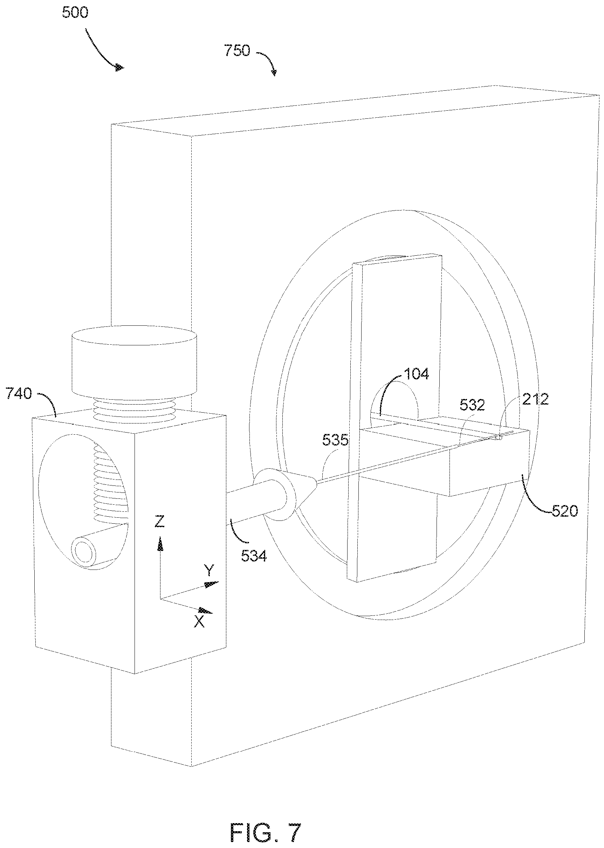

**FIG. 7** illustrates additional components of the sealant application system of **FIG. 5****.**

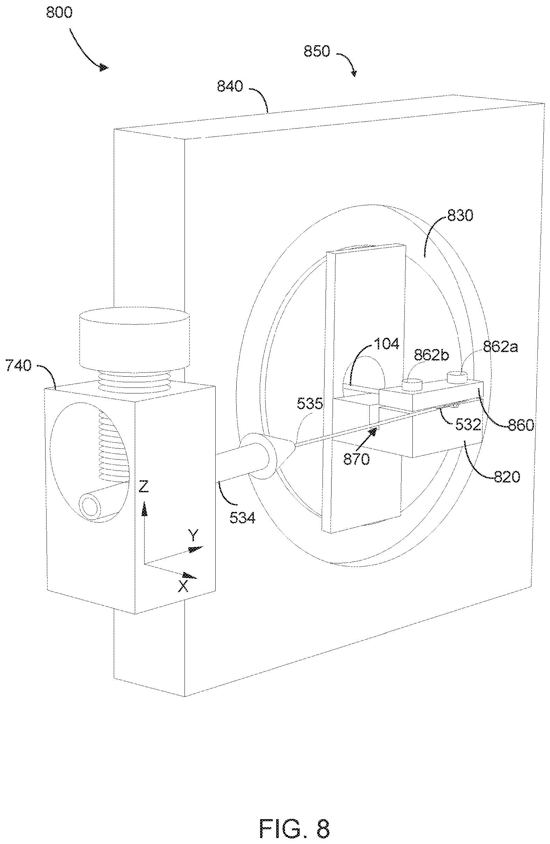

**FIG. 8** illustrates a sealant application system in accordance with a particular embodiment of the present invention.

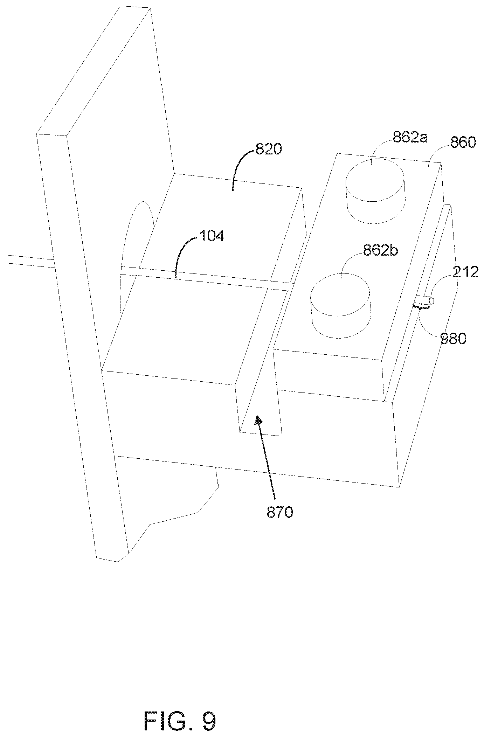

**FIG. 9** illustrates a cannula holder with a clamp in accordance with a particular embodiment of the present invention.

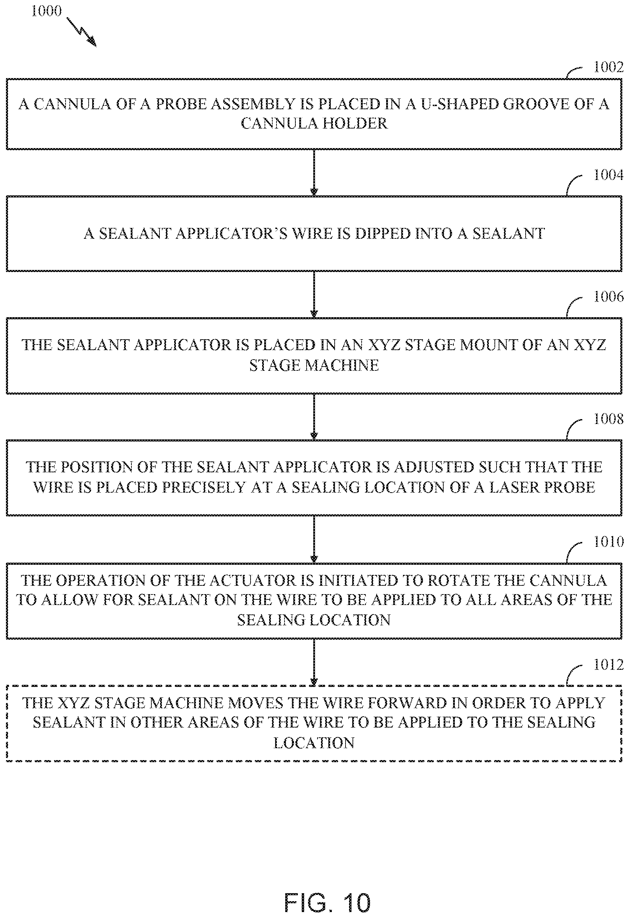

**FIG. 10** illustrates example operations for sealing a laser probe in accordance with a particular embodiment of the present invention.

To facilitate understanding, identical reference numerals have been used, where possible, to designate identical elements that are common to the drawings. It is contemplated that elements and features of one embodiment may be beneficially incorporated in other embodiments without further recitation.

### DETAILED DESCRIPTION

Aspects of the present disclosure provide methods and systems for manufacturing a thermally robust laser probe assembly.

As described above, a probe assembly with a high power throughput may experience overheating (e.g., when blood contaminates the lens or blocks the laser beam) such that the lens within the cannula may melt. A melting lens may also detach from the cannula resulting in the probe assembly malfunctioning.

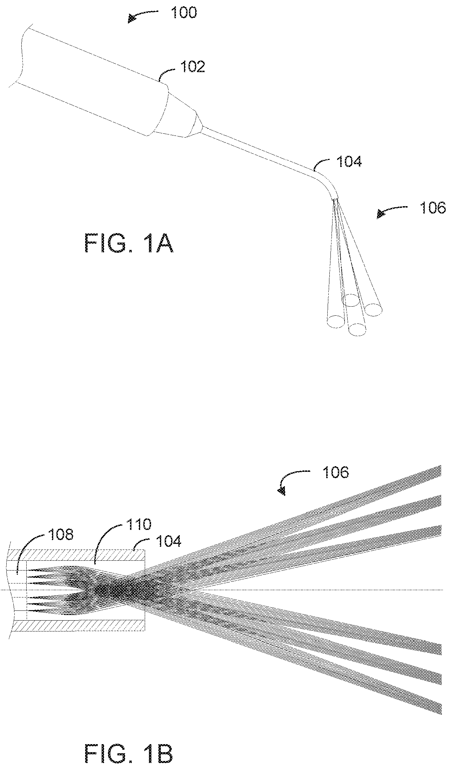

**FIG. 1A** illustrates an example of a probe assembly 100 comprising a hand-piece 102 and a cannula 104. A surgeon uses hand-piece 102 to guide cannula 104 (e.g., cylindrical shaped hollow tube) into a patient's body part, which may be a patient's eye. As shown, probe assembly 100 concurrently provides multiple photocoagulation beams 106 resulting in multiple laser spots. Each laser spot's power may be between about 250 and about 500 milliwatts (mW) such that by providing multiple laser spots, the minimum power passing through cannula 104 may be about 1 watt (W). A lens (e.g., lens 100 in **FIG. 1B**) may be placed in front of the optical fibers, which extend through the cannula, for projecting the laser beams onto, for example, a patient's eye's retinal surface.

**FIG. 1B** illustrates a cross-sectional view of the tip of cannula 104, where lens 110 is placed for collimating and projecting beams 106 propagated by multiple optical fibers 108 extending through cannula 104. Optical fibers 108, in certain embodiments of the present invention, may be an optical fiber array or a multi-core optical fiber. When cannula 104 is placed in a patient's body part, such as through a trocar cannula, beams 106 may be reflected back into cannula 104, such as when there is blood or other dark material in front of the tip of cannula 104 or partially blocking or touching lens 110. The reflection of laser beams back into cannula 104 adds to the amount of heat that is already generated within cannula 104. This overheating may melt cannula 104 and lens 110 and also cause lens 110 to detach from cannula 104.

In certain embodiments of the present invention, in order to protect lens 110, a protective component is attached to and/or inserted into, the distal end of a probe assembly's cannula. The protective component (e.g., protective window) is placed in front of the distal end of the lens that is itself placed in front of one or more optical fibers. The protective component protects the lens by restricting movements of the lens along the cannula and/or also by preventing the lens from detaching from the cannula.

**FIG. 2A** illustrates a cross-sectional view of an example protective component 212 that is placed at the tip of cannula 104. As shown, protective component 212 is placed at the distal end 205 of cannula 104 while the proximal end 207 of cannula 104 is connected to a hand-piece (e.g., hand-piece 102 shown in **FIG. 1A**). As described above, distal end 205 of cannula 104 is the end that is inserted into the patient's body part, or where laser light is configured to be emitted out of probe assembly 100. In certain embodiments, cannula 104 comprises material such as stainless steel, Nitinol (NiTi), or a platinum-iridium alloy (Pt-Ir).

Protective component 212 comprises proximal end 215 and distal end 213. In certain embodiments, protective component 212 comprises an optically clear or transparent material. Examples of suitable transparent materials include sapphire, fused silica, or other glass or ceramic materials with high transition temperatures.

In certain embodiments, protective component 212 is attached to cannula 104 by way of press-fitting component 212 into cannula 104. Press-fitting, also known as interference fitting or friction fitting, is a technique for securing protective component 212 to cannula 104, the securing being achieved by friction between protective component 212 and cannula 104 after protective component 212 is pushed into cannula 104. In certain embodiments, protective component 212 may be attached to cannula 104 using brazing techniques.

**FIG. 2B** illustrates a three-dimensional view of protective component 212. In certain embodiments, protective component 212 is a cylindrical component that is placed into the cylindrical opening in the distal end of cannula 104. Although protective component 212 shown in **FIGs. 2A-2B** is a cylindrical component with flat ends, in certain embodiments, protective component 212 may have a different shape. For example, in certain embodiments, the proximal end of protective component 212 may be spherical or aspherical.

**FIG. 2C** illustrates a front view of the tip of cannula 104 that houses protective component 212.

**FIG. 2D** illustrates a three-dimensional view of the tip of cannula 104. As shown, protective component 212 partially extends outside of cannula 104.

In certain cases, protective component 212 and cannula 104 do not have matching dimensional tolerances. Dimensional tolerances are assigned to parts, such as protective component 212 and cannula 104, as boundaries for acceptable build for manufacturing purposes. In situations where protective component 212 and cannula 104 do not have matching tolerances, the inner diameter of cannula 104 may be larger than the outer diameter of protective component 212 in certain areas, resulting in a gap. Also, in some situations, protective component 212 and cannula 104 may have surfaces with different degrees of roughness. Such incompatibilities and differences in dimensional tolerance and surface roughness may make the probe vulnerable to leaking. For example, fluids such as balanced salt solution (BSS), perfluorooctane (PFO), blood, etc., may leak into the cannula and reach in-between the lens and the fibers (e.g., lens 210 and fiber 108 of **FIG. 2A**), causing lens 210 and, therefore, probe assembly 100 to malfunction. As an example, the fluids leaking into cannula 104 may come in contact with anti-reflective-coated surfaces, resulting in lower laser beam transmittance, overheating the probe tip, and thermal runaway.

**FIG. 3** illustrates an example front view of cannula 104 housing protective component 212 in accordance with a particular embodiment of the present invention. In the example of **FIG. 3**, the inner diameter 330 of cannula 104 is larger than the outer diameter 332 of protective component 212 resulting in gap 334. As described above, this gap may result from incompatibilities and differences in dimensional tolerance and surface roughness between protective component 212 and cannula 104. Note that the size and shape of gap 334 are exemplary and also exaggerated for illustration purposes. In real applications, gap 334 may be, for example, much smaller than a micrometer. However, even a very small gap may make probe assembly 110 susceptible to leakage.

Accordingly, certain embodiments described herein relate to sealant application techniques for sealing openings or gaps in-between the inner diameter of the distal end of a cannula and the outer diameter of a protective component attached to the distal end of the cannula.

As the protective component and the cannula tip are of miniature size, applying a very small amount of sealant (e.g., adhesive material) accurately to the cannula and the protective component, at a sealing location described below, can be a challenge. Without a well-controlled sealant application technique, the protective component may be contaminated by sealant, resulting in blocking of the laser beam and a potential failure of the probe assembly. Also, excess sealant may be applied to the outer surface of the distal end of the cannula, resulting in an enlargement of the outer diameter of the distal end of the cannula, thereby, making it difficult for the surgeon to insert and remove the probe assembly's cannula into and from a trocar cannula that is inserted into the patient's body part.

**FIG. 4A** illustrates an example of sealant 402 contaminating protective component 212 and also overflowing onto the outer surface of the distal end of cannula 104. The sealant application techniques described herein overcome the challenge of applying a small amount of sealant to seal the probe without contaminating protective component 212 or overflowing onto the outer surface of cannula 104.

**FIG. 4B** illustrates an example cross sectional view of the distal end of cannula 104, which is sealed using the sealant application techniques described herein. As shown, sealant 406 is only applied to sealing location 430, which refers to any opening or gap (e.g., gap 334 in **FIG. 3**) between the inner surface of the distal end of cannula 104 and the outer surface of the distal end of the protective component 212. Therefore, as shown, sealant 406 has not contaminated protective component 212 or been applied to the outer surface of the distal end of cannula 104. In certain embodiments of the present invention, the sealant is a dual-cured sealant. Also, the sealant may have a viscosity in the range of about 500 to about 5000 centipoise (cP).

**FIG. 5A** illustrates an example sealant application system 500 for sealing the distal end of cannula 104 using the sealant application techniques described herein. As shown, system 500 comprises a cannula holder 520 having a u-shaped groove (e.g., groove 540 in **FIG. 5B**) where cannula 104 is able to be placed and rotated along an axis of rotation (e.g., axis of rotation 505 in **FIG. 5B**) parallel to cannula 104 itself. **FIG. 5B** illustrates a front view of cannula holder 520 holding cannula 104 in u-shaped groove 540. As shown, u-shaped groove 540 is configured such that a portion of cannula 104 remains above the stop surface of cannula holder 520. **FIG. 5B** also shows an axis of rotation 505 around which cannula 104 as well as protective component 212 are configured to rotate relative to cannula holder 520.

Referring back to **FIG. 5A****,** system 500 also comprises an XYZ stage machine (not shown) on which a sealant applicator 530 can be mounted. An XYZ stage machine is able to provide translations along the X, Y, and Z planes, as shown. Sealant applicator 530, as shown, comprises a hand-piece 534 with a rigid tube 535 and a wire 532 extending out of rigid tube 535. A user uses hand-piece 534 to dip wire 532 in sealant that is used during the sealing process. The user then places sealant applicator 530 on the XYZ stage machine and adjusts the position of the sealant applicator 530. Wire 532, in certain embodiments, is a very thin wire with a diameter in the range of about 20 to about 40 microns (µm). For example, wire 532 may have a diameter of about 40 µm. The very small diameter of wire 532 ensures that only a very small and controlled amount of sealant is picked-up by wire 532 when wire 532 is dipped in the sealant. In addition, in certain embodiments, wire 532 is flexible, which is advantageous because a flexible wire 532 may be able to flex in response to being pushed up or down against protective component 212 when the wire is being placed at the sealing location (e.g., sealing location 430 of **FIG. 4B**). A flexible wire 532 may also facilitate an even and smooth application of the sealant to the sealing location. As an example, wire 532 may be made of Nitinol.

Using the XYZ stage machine, wire 532 may be precisely placed at the sealing location, such that the sealant makes contact with both protective component 212 and cannula 104 at the sealing location. Once wire 532 is placed at the sealing location, cannula 104 may be rotated so that the sealant can be evenly distributed around and applied to the entire circumference of protective component 212 at the sealing location.

At the same time, as shown in **FIG. 6C**, sealant applicator 530 may also be moved to ensure that enough sealant is applied to the sealing location (e.g., to apply the additional sealant on other areas of wire 532 to the sealing location). Using a thin wire 532 ensures that the sealant is only applied to the sealing location and not to any additional areas of the protective component's surface. In other words, using a thin wire 532 allows for a more precise application of the sealant. Example stages of the sealant application procedure, in accordance with particular embodiments of the present invention, are illustrated in **FIGs. 6A-6D****.**

**FIG. 6A** illustrates wire 532 dipped in sealant as well as cannula 104 housing protective component 212. As described above, an XYZ stage machine may be used to precisely place wire 532 at sealing location 430.

**FIG. 6B** illustrates the tip of wire 532 being placed at sealing location 430. While not shown, once wire 532 is placed at sealing location 430, cannula 104 may be rotated (e.g., along axis of rotation 505 in FIG. 5B) so that the sealant can be evenly applied to the entire circumference of protective component 212 at the sealing location 430. After a certain number of rotations, however, there may not be enough sealant remaining on the tip of wire 532. As a result, as shown in **FIG. 6C**, wire 532 may be horizontally moved forward so that the sealant on the lower part of wire 532 can be used for the sealing.

**FIG. 6C** illustrates wire 532 being horizontally pushed forward (e.g., by an XYZ stage machine (not shown)) in order to apply the sealant on the lower part of wire 532 to sealing location 430. In another example, if the sealant on the lower part of wire 532 is first used, wire 532 may be pulled backward in order to use the sealant on the upper part of wire 532.

**FIG. 6D** illustrates an example sealing location 430 that is completely sealed using the sealant application technique described above.

**FIG. 7** illustrates additional components of the sealant application system 500 described in relation to **FIGS. 5A** and **5B****.** For example, **FIG. 7** illustrates an XYZ stage mount 740 that sealant applicator 530 is mounted on. XYZ stage 740 mount is able to be coupled to an XYZ stage machine (not shown) that is capable of moving XYZ stage mount 740 along the X, Y, and Z planes. As shown, using this mechanism, wire 532 is able to be placed precisely at the sealing location (e.g., sealing location 430 in **FIG. 4B**) and adjusted (e.g., pushed forward or backward) during the sealant application process. **FIG. 7** also provides a front view of a stage 750 that is coupled to an actuator (not shown) for rotating cannula 104 that is placed in the u-shaped groove (e.g., groove 540 in **FIG. 5B**) of cannula holder 520. In certain embodiments, the actuator is operated by a user, who is able to turn the actuator on and off. When the actuator is turned on, it rotates cannula 104 around an axis of rotation (e.g., axis of rotation 505 in **FIG. 5B**) parallel to cannula 104.

In certain embodiments, the duration for which cannula 104 is rotated and the speed at which cannula 104 is rotated are parameters that can be controlled and adjusted by a user. In certain other embodiments, the actuator is controlled and operated by a control module. In such embodiments, the control module operates the actuator for a certain number of rotations (e.g., or a certain amount of time) at a certain speed. The control module may also be configured to terminate the operation of the actuator after a user-defined number of rotations is performed.

In certain embodiments, a clamp may be used to further secure cannula 104 and prevent any undesired movements during the sealant application process. Using a clamp is especially advantageous when cannula 104 is not straight. For example, in some cases, a cannula's distal tip is curved as shown in **FIG. 1A****.** In such cases, the curved cannula, which may be made of elastic material such as Nitinol can be temporarily straightened and placed in the u-shaped groove of the cannula holder. The cannula can then be clamped such that the cannula tip is prevented from wobbling when it is rotated during the sealant application procedure.

**FIG. 8** illustrates an example sealant application system 800 for sealing a cannula with a curved tip. Although, note that system 800 may also be used for sealing a straight cannula as well. Sealant application system 800 comprises a rotation stage 850 including a stationary housing 840 and a rotatable wheel 830 that is coupled to cannula holder 820. Rotatable wheel 830 is configured to rotate cannula holder 820 along with cannula 104 (e.g., a curved cannula), which is clamped by clamp 860. In other words, unlike in **FIG. 7** where only cannula 104 is rotated during the sealant application procedure, in the embodiment illustrated in **FIG. 8****,** cannula 104 along with cannula holder 820 as well as rotatable wheel 830 are all rotated together around an axis of rotation parallel to cannula 104 (e.g., parallel to the X axis, as shown). In certain embodiments, rotatable wheel 830 may be coupled to an actuator that is configured to rotate rotatable wheel 830.

As shown in **FIG. 8****,** cannula holder 820 has an opening 870, which provides some hand-room, thereby, allowing a user to manipulate cannula 104 and adjust its placement, etc. In **FIG. 8****,** cannula holder 820 is shown as coupled to clamp 860 with screws 862a and 862b. Although, in other embodiments, elements other than screws may be used to couple clamp 860 to cannula holder 820.

**FIG. 9** illustrates another view of cannula holder 820 coupled to clamp 860 for clamping cannula 104. Clamp 860 is able to secure cannula 104 by applying pressure to the top of cannula 104. In certain embodiments, clamp 860 is made of flexible material such that clamping cannula 104 would not damage cannula 104. Note that, as described above, when cannula 104 is curved, the tip (e.g., shown as tip 980) of the distal end of cannula 104 may be left out of the u-shaped groove in order to prevent cannula 104 from wobbling during the rotations.

**FIG. 10** shows flowchart 1000, which illustrates the steps in a method for sealing a laser probe in accordance with a particular embodiment of the present invention. In some embodiments, the steps of flowchart 1000 are performed using a sealant application system (e.g., sealant application system 500). In certain embodiments, a user is involved in performing steps 1002-1012 and, in other embodiments, sealant application system 500 may be configured to automatically perform at least some of steps 1002-1012.

At step 1002, a cannula (e.g., cannula 104) of a probe assembly is positioned in a u-shaped groove of a cannula holder, such as by a user. As described in relation to **FIG. 2A****,** a protective component is placed at the distal end of the cannula. At its proximal end, the cannula is coupled to an actuator for rotating the cannula.

At step 1004, a sealant applicator's wire is dipped into a sealant.

At step 1006, the sealant applicator is placed in an XYZ stage mount of an XYZ stage machine.

At step 1008, the position of the sealant applicator is adjusted such that a wire of the sealant applicator is placed at a sealing location of a laser probe. Placing the wire at the sealing location causes the sealant on the wire to be applied to at least an area of the sealing location (e.g., sealing location 430 of **FIG. 4**).

At step 1010, the operation of the actuator to rotate the cannula is initiated. Rotating the cannula allows for the sealant on the wire to be applied to all areas of the sealing location.

At step 1012, optionally the XYZ stage machine moves the wire forward in order to apply sealant in other areas (e.g., lower areas) of the wire to the sealing location. The actuator continues to rotate the cannula until the sealing location is completely sealed.

Performing steps 1002 through 1010 (and optionally 1012) of flowchart 1000 results in a thermally robust laser probe assembly with a sealed distal end that prevents or, at least, reduces the amount of fluids that may leak into the probe. In certain embodiments, after the sealing location is sealed, the probe may be dual-cured (ultraviolet (UV) + thermal curing of the adhesive).

The foregoing description is provided to enable any person skilled in the art to practice the various embodiments described herein. Various modifications to these embodiments will be readily apparent to those skilled in the art, and the generic principles defined herein may be applied to other embodiments. Thus, the claims are not intended to be limited to the embodiments shown herein, but are to be accorded the full scope consistent with the language of the claims.

## Claims

1. A method of manufacturing a probe assembly, the probe assembly including a cannula (104) through which one or more optical fibers extend at least partially for transmitting laser light from a laser source to a target location, a lens (210) housed in the cannula, and a protective component (212) at a distal end of the cannula, wherein the lens (210) is positioned between the one or more optical fibers (108) and the protective component, to provide a probe assembly having a sealed distal end, **characterised in that** the method comprises :
positioning (1002, 1004) a wire (532) coated with sealant (406) at a sealing location at the distal end of the cannula (104) of the probe assembly;
rotating the cannula (1010) to apply the sealant (406) to the sealing location;
wherein the sealant (406) is applied to gaps (334) resulting from incompatibilities and differences in dimensional tolerance and surface roughness between the outer surface of the protective component and the inner surface of the cannula.

2. The method of claim 1, further comprising:
moving (1012) the wire (532) forward and backward during the rotating.

3. The method of claim 1, further comprising:
dual-curing the sealant after application.

4. The method of claim 1 comprising providing a sealant application system (500), wherein the sealant application system comprises:
a sealant applicator (530) comprising the wire (532),
a stage machine (740) configured to hold the sealant applicator (530) and to position the wire at the distal end of a cannula (104) of a probe assembly; and
an actuator configured to rotate (1010) the cannula once the stage machine has positioned the wire at the distal end thereof such that sealant on the wire applied thereto.

5. The method of claim 1 wherein a diameter of the wire (532) is within a range of about 20 to about 40 micrometers.

6. The method of claim 1 further comprising:
positioning the cannula of the probe assembly, wherein the protective component is placed at the distal end of the cannula, and wherein a proximal end of the cannula is coupled to the actuator for rotating the cannula.

7. The method of claim 6 further comprising:
dipping the wire of the sealant applicator into the sealant.

8. The method of claim 7 further comprising:
placing the sealant applicator in an XYZ stage mount of an XYZ stage machine.

9. The method of claim 8 further comprising:
adjusting, the position of the sealant applicator such that the wire of the sealant applicator is placed at the sealing location at the distal end of the probe.

10. The method of claim 9 further comprising:
operating of the actuator to rotate the cannula wherein rotating the cannula allows for the sealant on the wire to be applied to all areas of the sealing location.

11. The method of claim 10 further comprising:
operating the XYZ stage machine to move the wire forward to apply the sealant in other areas of the wire to the sealing location wherein the actuator continues to rotate the cannula until the sealing location is completely sealed.

12. The method of claim 11 further comprising:
dual curing the probe after the sealant is applied.

## Patentansprüche

1. Verfahren zum Herstellen einer Sondenbaugruppe, wobei die Sondenbaugruppe eine Kanüle (104), durch die sich eine oder mehrere optische Fasern wenigstens teilweise zum Transmittieren von Laserlicht von einer Laserquelle zu einem Zielort erstrecken, eine Linse (210), die in der Kanüle untergebracht ist, und eine Schutzkomponente (212) an einem distalen Ende der Kanüle beinhaltet, wobei die Linse (210) zwischen der einen oder den mehreren optischen Fasern (108) und der Schutzkomponente positioniert ist, um eine Sondenbaugruppe mit einem abgedichteten distalen Ende bereitzustellen, **dadurch gekennzeichnet, dass** das Verfahren Folgendes umfasst:
Positionieren (1002, 1004) eines Drahtes (532), der mit einem Dichtungsmittel (406) beschichtet ist, an einer Dichtungsstelle an dem distalen Ende der Kanüle (104) der Sondenbaugruppe;
Drehen der Kanüle (1010), um das Dichtungsmittel (406) an der Dichtungsstelle aufzubringen;
wobei das Dichtungsmittel (406) auf Spalte (334) aufgebracht wird, die aus Inkompatibilitäten und Unterschieden der Abmessungstoleranz und Oberflächenrauhigkeit zwischen der Außenoberfläche der Schutzkomponente und der Innenoberfläche der Kanüle resultieren.

2. Verfahren nach Anspruch 1, das ferner Folgendes umfasst:
Bewegen (1012) des Drahts (532) vor und zurück während des Drehens.

3. Verfahren nach Anspruch 1, das ferner Folgendes umfasst:
duales Aushärten des Dichtungsmittels nach dem Aufbringen.

4. Verfahren nach Anspruch 1, das Bereitstellen eines Dichtungsmittelaufbringungssystems (500) umfasst, wobei das Dichtungsmittelaufbringungssystem Folgendes umfasst:
einen Dichtungsmittelapplikator (530), der den Draht (532) umfasst,
eine Positionierungseinheit (740), die dazu konfiguriert ist, den Dichtungsmittelapplikator (530) zu halten und den Draht am distalen Ende einer Kanüle (104) einer Sondenbaugruppe zu positionieren; und
einen Aktuator, der dazu konfiguriert ist, die Kanüle zu drehen (1010), sobald die Positionierungseinheit den Draht an dem distalen Ende davon positioniert hat, so dass Dichtungsmittel auf dem Draht darauf aufgebracht wird.

5. Verfahren nach Anspruch 1, wobei ein Durchmesser des Drahtes (532) innerhalb eines Bereichs von etwa 20 bis etwa 40 Mikrometer liegt.

6. Verfahren nach Anspruch 1, das ferner Folgendes umfasst:
Positionieren der Kanüle der Sondenbaugruppe, wobei die Schutzkomponente an dem distalen Ende der Kanüle platziert ist und wobei ein proximales Ende der Kanüle mit dem Aktuator zum Drehen der Kanüle gekoppelt ist.

7. Verfahren nach Anspruch 6, das ferner Folgendes umfasst:
Eintauchen des Drahtes des Dichtungsmittelapplikators in das Dichtungsmittel.

8. Verfahren nach Anspruch 7, das ferner Folgendes umfasst:
Platzieren des Dichtungsmittelapplikators in einer XYZ-Positionierungshalterung einer XYZ-Positionierungsmaschine.

9. Verfahren nach Anspruch 8, das ferner Folgendes umfasst:
Anpassen der Position des Dichtungsmittelapplikators derart, dass der Draht des Dichtungsmittelapplikators an der Dichtungsstelle am distalen Ende der Sonde platziert wird.

10. Verfahren nach Anspruch 9, das ferner Folgendes umfasst:
Betreiben des Aktuators zum Drehen der Kanüle, wobei das Drehen der Kanüle ermöglicht, dass das Dichtungsmittel auf dem Draht auf alle Bereiche der Dichtungsstelle aufgebracht wird.

11. Verfahren nach Anspruch 10, das ferner Folgendes umfasst:
Betreiben der XYZ-Positionierungsmaschine dahingehend, den Draht vorwärts zu bewegen, um das Dichtungsmittel in anderen Bereichen des Drahtes auf die Dichtungsstelle aufzubringen, wobei der Aktuator die Kanüle weiterhin dreht, bis die Dichtungsstelle vollständig abgedichtet ist.

12. Verfahren nach Anspruch 11, das ferner Folgendes umfasst:
duales Aushärten der Sonde nach dem Aufbringen des Dichtungsmittels.

## Revendications

1. Procédé de fabrication d'un ensemble sonde, l'ensemble sonde comprenant une canule (104) à travers laquelle une ou plusieurs fibres optiques s'étendent au moins partiellement pour transmettre la lumière laser d'une source laser à un emplacement cible, une lentille (210) logée dans la canule, et un composant de protection (212) à une extrémité distale de la canule, la lentille (210) étant positionnée entre la ou les fibres optiques (108) et le composant de protection, pour fournir un ensemble sonde ayant une extrémité distale scellée, **caractérisé en ce que** le procédé comprend :
le positionnement (1002, 1004) d'un fil (532) recouvert de produit d'étanchéité (406) au niveau d'un emplacement de scellement au niveau de l'extrémité distale de la canule (104) de l'ensemble sonde ;
la rotation de la canule (1010) pour appliquer le produit d'étanchéité (406) sur l'emplacement de scellement ;
le produit d'étanchéité (406) étant appliqué à des espaces (334) résultant d'incompatibilités et de différences de tolérance dimensionnelle et de rugosité de surface entre la surface externe du composant de protection et la surface interne de la canule.

2. Procédé selon la revendication 1, comprenant en outre :
le déplacement (1012) du fil (532) vers l'avant et vers l'arrière pendant la rotation.

3. Procédé selon la revendication 1, comprenant en outre :
le double durcissement du produit d'étanchéité après application.

4. Procédé selon la revendication 1, comprenant la fourniture d'un système d'application de produit d'étanchéité (500), le système d'application de produit d'étanchéité comprenant :
un applicateur de produit d'étanchéité (530) comprenant le fil (532),
une machine à étages (740) configurée pour maintenir l'applicateur de produit d'étanchéité (530) et pour positionner le fil au niveau de l'extrémité distale d'une canule (104) d'un ensemble sonde ; et
un actionneur configuré pour faire tourner (1010) la canule une fois que la machine à étages a positionné le fil à son extrémité distale de sorte que le produit d'étanchéité sur le fil lui est appliqué.

5. Procédé selon la revendication 1, le diamètre du fil (532) étant compris dans une plage d'environ 20 à environ 40 micromètres.

6. Procédé selon la revendication 1, comprenant en outre :
le positionnement de la canule de l'ensemble sonde, le composant de protection étant placé à l'extrémité distale de la canule, et une extrémité proximale de la canule étant couplée à l'actionneur pour faire tourner la canule.

7. Procédé selon la revendication 6, comprenant en outre :
le trempage du fil de l'applicateur de produit d'étanchéité dans le produit d'étanchéité.

8. Procédé selon la revendication 7, comprenant en outre :
le placement de l'applicateur de produit d'étanchéité dans un support de platine XYZ d'une machine à étages XYZ.

9. Procédé selon la revendication 8, comprenant en outre :
le réglage de la position de l'applicateur de produit d'étanchéité de telle sorte que le fil de l'applicateur de produit d'étanchéité soit placé au niveau de l'emplacement de scellement à l'extrémité distale de la sonde.

10. Procédé selon la revendication 9, comprenant en outre :
l'actionnement de l'actionneur pour faire tourner la canule, la rotation de la canule permettant d'appliquer le produit d'étanchéité sur le fil sur toutes les zones de l'emplacement de scellement.

11. Procédé selon la revendication 10, comprenant en outre :
l'utilisation de la machine à étages XYZ pour déplacer le fil vers l'avant afin d'appliquer le produit d'étanchéité dans d'autres zones du fil à l'emplacement de scellement l'actionneur continuant à faire tourner la canule jusqu'à ce que l'emplacement de scellement soit complètement scellé.

12. Procédé selon la revendication 11, comprenant en outre :
le double durcissement de la sonde après l'application du produit d'étanchéité.
